Europäisches Patentamt

European Patent Office

Office européen des brevets

(19)

(11) Numéro de publication: **0 273 201 B1**

(12)

## FASCICULE DE BREVET EUROPEEN

(45) Date de publication de fascicule du brevet:
17.04.91

(51) Int. Cl.⁵: **A61M 5/14**, A61M 1/36,
A61K 35/14

(21) Numéro de dépôt: 87117391.0

(22) Date de dépôt: 25.11.87

(54) Réchauffeur rapide pour sang et produits sanguins.

(30) Priorité: 28.11.86 FR 8616859

(43) Date de publication de la demande:
06.07.88 Bulletin 88/27

(45) Mention de la délivrance du brevet:
17.04.91 Bulletin 91/16

(84) Etats contractants désignés:
AT BE CH DE ES FR GB GR IT LI LU NL SE

(56) Documents cités:
DE-A- 2 515 889
DE-A- 3 502 095
FR-A- 1 140 895
US-A- 3 064 649

(73) Titulaire: **Nicolas Marchiani Chatelain, Marie-Louise**
**Le Lesdiguière Résidence St. Mury**
**F-38240 Meylan(FR)**

Titulaire: **Pellini, Jean-Pierre**
**Tèche**
**F-38470 Vinay(FR)**

(72) Inventeur: **Nicolas Marchiani Chatelain, Marie-Louise**
**Le Lesdiguière Résidence St. Mury**
**F-38240 Meylan(FR)**
Inventeur: **Pellini, Jean-Pierre**
**Tèche**
**F-38470 Vinay(FR)**

(74) Mandataire: **Casalonga, Axel et al**
**BUREAU D.A. CASALONGA - JOSSE Morassistrasse 8**
**W-8000 München 5(DE)**

Rank Xerox (UK) Business Services

## Description

La présente invention concerne un dispositif selon le préambule de la rev. 1 pour réchauffer ou décongeler des produits d'injection ou de perfusion, en particulier des produits sanguins, contenus dans des poches ou des flacons hernétiquement fermés, afin d'en permettre l'injection ou la transfusion notamment lors d'une grande déperdition sanguine au cours d'une intervention chirurgicale en bloc opératoire.

Le réchauffement ou la décongélation notamment de produits sanguins sont généralement effectués en les mettant dans un récipient que l'on remplit d'eau plus ou moins chaude, sans contrôle de la température, en renouvelant plusieurs fois l'opération. Ce procédé est lent et présente des risques d'altération des produits. Une autre méthode consiste à utiliser un réchauffeur électrique au travers duquel passe une tubulure dans laquelle circule les produits à réchauffer. Lorsque la vitesse de transfusion doit être rapide, les produits passant dans le réchauffeur électrique n'ont pas le temps de se réchauffer. Dans le brevet DE-A-3 502 095, il est décrit un réchauffeur dans lequel la poche à réchauffer est disposée dans une cuve contenant de l'eau chaude a température réglable.

La présente invention vise à remédier aux inconvénients ci-dessus et propose un dispositif qui, bien qu'étant très simple, permet de réchauffer ou décongeler, dans une cuve, des produits d'injection ou de perfusion contenus dans des poches ou flacons hermétiquement fermés, en particulier des produits sanguins, rapidement et sans risque d'altération de ces derniers.

Le dispositif conforme à la présente invention comprend des moyens permettant d'introduire en continu de l'eau mitigée dans la cuve précitée et présentant au moins un conduit d'amenée sur lequel sont montés un robinet thermostatique réglable connecté d'une part au réseau de distribution d'eau froide et d'autre part au réseau de distribution d'eau chaude et une vanne de réglage de débit, la cuve étant munie à sa partie supérieure d'au moins un déversoir pour l'écoulement de l'eau, tel qu'au moins une poche ou un flacon de produits disposé dans la cuve est immergée, l'eau mitigée circulant autour de cette poche ou de ce flacon.

De préférence, ledit conduit d'amenée d'eau mitigée débouche dans la partie inférieure de ladite cuve. La sortie de ce conduit ledit déversoir peuvent être avantageusement situés en des endroits opposés de ladite cuve.

Le dispositif selon à la présente invention, comprend de préférence un conduit de vidange de ladite cuve sur lequel est monté une vanne. En outre, ledit déversoir est de préférence constitué par un conduit d'écoulement relié à ce conduit de vidange.

Conformément à la présente invention, lesdits moyens d'amenée d'eau mitigée et lesdits conduits sont de préférence montés sur ladite cuve de manière à former un ensemble, cet ensemble comprenant des moyens en vue de sa fixation.

Le dispositif selon la présente invention, porte de préférence un afficheur permettant d'indiquer la nature des produits introduits dans ladite cuve ainsi que des moyens pouvant recevoir la carte signalétique du patient auquel sont destinés ces produits.

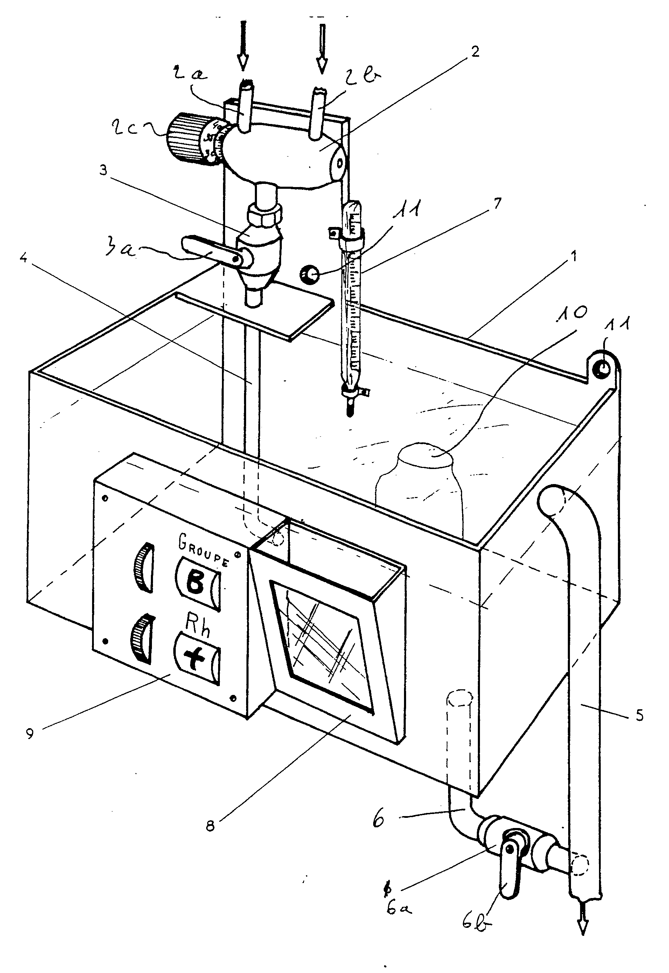

La présente invention sera mieux comprise à l'étude d'un dispositif pour réchauffer ou décongeler des produits d'injection ou de perfusion, en particulier des produits sanguins, contenus dans des poches ou des flacons hermétiquement fermés, décrit à titre d'exemple non limitatif et illustré sur la figure unique annexée.

Le dispositif représenté sur la figure comprend une cuve 1 de préférence en acier inoxydable et de forme parallélipipédique. Cette cuve 1 peut être alimentée ou remplie en eau mitigée par l'intermédiaire d'un robinet thermostatique 2 réglable grâce à un bouton 2c par exemple jusqu'à 40° maximum et d'une vanne 3 de réglage de débit. Ce robinet 2 et cette vanne 3 sont, dans l'exemple, successivement montés sur un conduit vertical 4 qui descend dans la cuve 1 jusqu'au voisinage de son fond, le robinet thermostatique 2 et la vanne 3 étant au-dessus de la cuve et étant montés sur une plaque la prolongeant vers le haut la paroi arrière de la cuve 1. Le robinet thermostatique 2 est connecté, par ses deux entrées 2a et 2b, d'une part au réseau de distribution d'eau froide et d'autre part au réseau de distribution d'eau chaude du bâtiment ou de la salle dans laquelle le dispositif est installé. L'eau introduite dans la cuve 1 peut en ressortir par l'intermédiaire d'un déversoir constitué dans l'exemple par un conduit vertical d'évacuation 5 dont l'extrémité supérieure coudée débouche dans la cuve 1 à sa partie supérieure, sa position déterminant le niveau de l'eau dans la cuve 1.

La cuve 1 est également munie à sa partie inférieure d'un conduit de vidange 6 sur lequel est montée une vanne 6a munie d'un bouton 6b. Le conduit 5 et le conduit 6 sont par ailleurs reliés entre eux pour évacuer l'eau par exemple vers le réseau d'évacuation du bâtiment, de préférence par l'intermédiaire d'un syphon non représenté.

Le dispositif décrit ci-dessus fonctionne de la manière suivante. La vanne 6a du conduit de vidange 6 étant fermée, on règle le bouton 2c de réglage de température du robinet thermostatique 2 à la température désirée et on ouvre la vanne 3. L'eau mitigée à la température désirée obtenue par le mélange de l'eau froide provenant du réseau de distribution d'eau froide par le conduit 2a et de l'eau chaude provenant du réseau de distribution d'eau chaude par le conduit 2b arrive dans le fond de la cuve 1 par l'intermédiaire du conduit vertical 4, sans provoquer d'éclaboussures. On règle le débit de l'eau introduite dans la cuve par la vanne 3.

Lorsque le niveau de l'eau mitigée atteint l'entrée supérieure du conduit 5, le trop plein est évacué par ce conduit. De la sorte, l'eau mitigée circule en continu dans la cuve 1 au débit réglé par la vanne 3. La sortie du conduit d'amenée d'eau mitigée 4 et l'entrée supérieure du conduit d'évacuation 5 étant situées en des endroits opposés de la cuve 1, l'eau ne stagne dans aucune partie de la cuve 1.

De préférence lorsque la température de l'eau circulant dans la cuve 1 est à une valeur déterminée, cette température étant contrôlable par l'intermédiaire d'un thermomètre 7 placé verticalement, on introduit dans la cuve 1 le flacon ou la poche 10 de produits d'injection ou de perfusion, en particulier de produits sanguins que l'on veut réchauffer ou décongeler, dans la cuve 1 de telle sorte que ce dernier se trouve de préférence complètement immergé, l'eau mitigée circulant en continu autour du flacon ou de la poche 10. Lorsque le produit contenu dans le flacon ou la poche 10 est réchauffé ou décongelé, on l'extrait et on peut alors utiliser les produits. De préférence, après chaque opération, on ferme la vanne d'entrée 3 et on ouvre la vanne de vidange 6a de manière à vider complètement la cuve 1 de l'eau qu'elle contient.

On voit sur la face avant de la cuve 1 que sont prévus d'une part un réceptable 8 dans lequel peut être introduite la carte signalétique du patient auquel sont destinés les produits que l'on réchauffe ou décongèle ainsi qu'un afficheur 9 à rouleaux permettant d'afficher le groupe et le rhésus sanguin du patient.

Le dispositif qui vient d'être décrit constitue un ensemble susceptible d'être fixé par exemple à une paroi par des vis traversant des orifices 11 de la partie supérieure de la paroi arrière de la cuve 1.

La présente invention ne se limite pas à l'exemple de réalisation ci-dessus décrit. Bien des variantes de réalisation sont possibles sans sortir du cadre défini par les revendications annexées.

**Revendications**

1. Dispositif pour réchauffer ou décongeler des produits d'injection ou de perfusion, en particulier les produits sanguins, contenus dans des poches ou des flacons (10) hermétiquement fermés dans lequel les poches ou flacons sont disposés dans une cuve (1), caractérisé par le fait qu'il comprend des moyens permettant d'introduire en continu de l'eau mitigée dans ladite cuve et présentant au moins un conduit d'amenée (4) sur lequel sont montés un robinet thermostatique (2) réglable connecté d'une part au réseau de distribution d'eau froide (2a) et d'autre part au réseau de distribution d'eau chaude (2b) et une vanne (3) de réglage de débit, la cuve étant munie à sa partie supérieure d'au moins un déversoir (5) pour l'écoulement de l'eau, tel qu'au moins une poche ou un flacon (10) de produit disposé dans la cuve (1) est immergée, l'eau mitigée circulant autour de cette poche ou de ce flacon.

2. Dispositif selon la revendication 1, caractérisé par le fait que ledit conduit d'emenée (4) d'eau mitigée débouche dans la partie inférieure de ladite cuve (1).

3. Dispositif selon l'une des revendications 1 et 2, caractérisé par le fait que la sortie dudit conduit d'emenée (4) d'eau mitigée et ledit déversoir (5) sont situés en des endroits opposés de ladite cuve (1).

4. Dispositif selon l'une quelconque des revendications précédentes, caractérisé par le fait qu'il comprend un conduit de vidange (6) de ladite cuve (1) sur lequel est monté une vanne (6a).

5. Dispositif selon l'une quelconque des revendications précédentes, caractérisé par le fait que ledit déversoir est constitué par un conduit d'écoulement (5) relié à un conduit de vidange (6) de ladite cuve sur lequel est montée une vanne (6a).

6. Dispositif selon l'une quelconque des revendications précédentes, caractérisé par le fait qu'il comprend un thermomètre (7) pour le contrôle de la température de l'eau circulant dans ladite cuve (1).

7. Dispositif selon l'une quelconque des revendications précédentes, caractérisé par le fait que lesdits moyens d'amenée d'eau mitigée et lesdits conduits sont montés sur ladite cuve (1) de manière à former un ensemble, cet ensemble comprenant des moyens en vue de sa fixation.

8. Dispositif selon l'une quelconque des revendications précédentes, caractérisé par le fait qu'il porte un afficheur (9) permettant d'indiquer les caractéristiques des produits introduits dans ladite cuve (1) ainsi que des moyens (8) pouvant recevoir la carte signalétique du patient auquel sont destinés ces produits.

## Claims

1. Device for heating or thawing injection or infusion products, especially blood products, contained in hermetically sealed bags or glass bottles (10), where the bags or bottles are arranged in a case (1), characterized in that it comprises means making it possible to continuously supply mixed water to said case and that it is equipped with at least one feeding pipe (4) provided with a controllable thermostatic valve (2) and connected to the cold water circuit (2a) as well as the hot water circuit (2b) and a flow governor (3); the upper part of the case being equipped with at least one overflow (5) for water outlet, which is installed in such a way that at least one bag or one glass bottle (10) of product in the case (1) is immersed in the water, whereby the mixed water circulates around the bag or the glass bottle.

2. The device of claim 1 characterized by said feeding pipe (4) for mixed water leading into the bottom of said case (1).

3. The device of claim 1 or 2, characterized by the outflow of said feeding pipe (4) for mixed water and said overflow (5) being installed at opposite sides of said case (1).

4. The device of one of the preceding claims, characterized in that it comprises a drain pipe (6) for said case (1), a drain pipe to which a valve (6a) is connected.

5. The device of one of the preceding claims, characterized by said overflow consisting of an outflow pipe (5) which is connected to the drain pipe (6) of said case, and to which a valve is connected (6a).

6. The device of one of the preceding claims, characterized in that it comprises a thermometer (7) for checking the temperature of the water circulating in said case (1).

7. The device of one of the preceding claims, characterized in that said feeding means for mixed water and said pipe are mounted at said case (1), thus, forming an entity, provided with fastening means.

8. The device of one of the preceding claims, characterized in that it is equipped with an indicator (9) making it possible to indicate the characteristics of the product in said case (1) as well as a holding device (8) for the identification card of the patient to receive the product.

## Ansprüche

1. Vorrichtung zum Anwärmen oder Auftauen von Injektions- oder Infusionsprodukten, insbesondere Blutprodukten in dicht verschlossenen Beuteln oder Glasflaschen (10), in welcher die Beutel bzw. Glasflaschen in einem kastenförmigen Behälter (1) angeordnet sind, dadurch gekennzeichnet, daß kontinuierliches Zuführen von Mischwasser in den kastenförmigen Behälter durch entsprechende Einrichtungen ermöglicht wird und mindestens eine Zuführleitung (4) vorhanden ist, die mit einem regelbaren Thermostatventil (2) versehen ist, das sowohl an die Kaltwasserleitung (2a) als auch an die Warmwasserleitung (2b) und an ein Durchlaufregelventil (3) angeschlossen ist, daß am oberen Teil des kastenförmigen Behälters mindestens ein Oberlauf (5) für den Ablauf des Wassers so angebracht worden ist, daß zumindest ein mit Produkt gefüllter Beutel bzw. eine mit Produkt gefüllte Glasflasche (10) im kastenförmigen Behälter (1) mit Wasser bedeckt ist, wobei das Mischwasser um den Beutel bzw. die Flasche zirkuliert.

2. Vorrichtung gemäß Anspruch 1, dadurch gekennzeichnet, daß der Zulauf (4) für Mischwasser in den unteren Teil des kastenförmigen Behälters (1) mündet.

3. Vorrichtung gemäß Anspruch 1) oder 2), dadurch gekennzeichnet, daß die Austrittsöftnungen des Mischwasserzulaufs (4) und des Üherlaurs (5) so am kastenförmigen Behälter (1) befestigt sind, daß sie einander gegenüberliegen.

4. Vorrichtung gemäß eines der vorangehenden Ansprüche, dadurch gekennzeichnet, daß für den kastenförmigen Behälter (1) eine Entleerungsleitung (6) vorhanden ist, die mit einem Ventil ausgestattet ist (6a).

5. Vorrichtung gemäß eines der vorangehenden Ansprüche, dadurch gekennzeichnet, daß der überlauf aus einem Ablaufrohr (5) besteht, das mit dem Entleerungsrohr (6) des kastenförmigen Behälters verbunden ist, welches mit einem Ventil (6a) versehen ist.

6. Vorrichtung gemäß eines der' vorangehenden Ansprüche, dadurch gekennzeichnet, daß diese mit einem Thermometer (7) ausgestattet ist, zur Temperaturüberwachung des Wassers, das in dem kastenförmigen Behälter zirkuliert (1).

7. Vorrichtung gemäß eines der vorangehenden Ansprüche, dadurch gekennzeichnet, daß die Zuführvorrichtungen für Mischwasser und die Leitungen auf dem kastenförmigen Behälter (1) angebracht sind, und somit eine Einheit bilden, welche mit Befestigungsvorrichtungen versehen ist.

8. Vorrichtung gemäß eines der vorangehenden Ansprüche, dadurch gekennzeichnet, daß es eine Anzeige (9) besitzt, mit deren Hilfe die Daten des im Behälter (1) befindlichen Produkts angezeigt werden können, sowie eine Vorrichtung (8) für die Kennkarte des Patienten, für den diese Produkte bestimmt sind.